# EUROPEAN PATENT SPECIFICATION

(11) **EP 2 048 241 B1**
(45) Date of publication and mention of the grant of the patent: **25.07.2012**
(21) Application number: 07019663.9
(22) Date of filing: 08.10.2007
(51) Int. Cl.: C12Q 1/68, G01N 33/574

(54) **Method employing GAPDH as molecular markers for cancer prognosis**
GAPDH als molekulare Marker zur Krebsprognose verwendende Verfahren
Procédé utilisant le GAPDH comme marqueur moléculaire pour le pronostic du cancer

(43) Date of publication of application: 15.04.2009
(73) Proprietor: Sividon Diagnostics GmbH, 50829 Köln (DE)
(72) Inventor: Stropp, Udo, Dr., 42781 Haan (DE); Wirtz, Ralph, Dr., 50677 Köln (DE); von Törne, Christian, Dr., 42659 Solingen (DE)
(74) Representative: von Kreisler Selting Werner

(56) References cited:
- WO-A-01/62286
- WO-A-2005/083429
- WO-A2-2007/016367
- US-A1- 2003 129 653
- RONDINELLI R H ET AL: "INCREASED GLYCERALDEHYDE-3-PHOSPHATE DEHYDROGENASE GENE EXPRESSION IN LATE PATHOLOGICAL STAGE HUMAN PROSTATE CANCER" PROSTATE CANCER AND PROSTATIC DISEASES, STOCKTON PRESS, BASINGSTOKE,, GB, vol. 1, no. 2, December 1997 (1997-12), pages 66-72, XP009024855 ISSN: 1365-7852
- OHL FALK ET AL: "Gene expression studies in prostate cancer tissue: which reference gene should be selected for normalization?" JOURNAL OF MOLECULAR MEDICINE (BERLIN, GERMANY) DEC 2005, vol. 83, no. 12, December 2005 (2005-12), pages 1014-1024, XP002472573 ISSN: 0946-2716
- EPNER D E ET AL: "Association of glyceraldehyde-3-phosphate dehydrogenase expression with cell motility and metastatic potential of rat prostatic adenocarcinoma." CANCER RESEARCH 1 MAY 1993, vol. 53, no. 9, 1 May 1993 (1993-05-01), pages 1995-1997, XP002472527 ISSN: 0008-5472
- LU S ET AL: "Identification of an additional hypoxia responsive element in the glyceraldehyde-3-phosphate dehydrogenase gene promoter" BIOCHIMICA ET BIOPHYSICA ACTA . GENE STRUCTURE AND EXPRESSION, ELSEVIER, AMSTERDAM, NL, vol. 1574, no. 2, 19 March 2002 (2002-03-19), pages 152-156, XP004349001 ISSN: 0167-4781
- DESPREZ P Y ET AL: "GLYCERALDEHYDE-3-PHOSPHATE DEHYDROGENASE GAPDH EC 1.2.1.12 GENE EXPRESSION IN TWO MALIGNANT HUMAN MAMMARY EPITHELIAL CELL LINES BT-20 AND MCF-7 REGULATION OF GENE EXPRESSION BY 1 25 DIHYDROXYVITAMIN D-3 1 25-OH-2D-3" CANCER LETTERS, vol. 64, no. 3, 1992, pages 219-224, XP002472528 ISSN: 0304-3835
- BONAFÉ NATHALIE ET AL: "Glyceraldehyde-3-phosphate dehydrogenase binds to the AU-Rich 3' untranslated region of colony-stimulating factor-1 (CSF-1) messenger RNA in human ovarian cancer cells: possible role in CSF-1 posttranscriptional regulation and tumor phenotype." CANCER RESEARCH 1 MAY 2005, vol. 65, no. 9, 1 May 2005 (2005-05-01), pages 3762-3771, XP002472529 ISSN: 0008-5472
- KOSTADIMA LIDA ET AL: "Survivin and glycodelin transcriptional activity in node-positive early breast cancer: mRNA expression of two key regulators of cell survival" BREAST CANCER RESEARCH AND TREATMENT, vol. 100, no. 2, November 2006 (2006-11), pages 161-167, XP002472531 ISSN: 0167-6806
- BHATIA PARDEEP ET AL: "Comparison of glyceraldehyde-3-phosphate dehydrogenase and 28S-ribosomal RNA gene expression as RNA loading controls for northern blot analysis of cell lines of varying malignant potential" ANALYTICAL BIOCHEMISTRY, vol. 216, no. 1, 1994, pages 223-226, XP002472571 ISSN: 0003-2697
- GERARD ET AL: "Mitochondrial ATP synthase 6 as an endogenous control in the quantitative RT-PCR analysis of clinical cancer samples" MOLECULAR DIAGNOSIS, NAPERVILLE, IL, US, vol. 5, no. 1, 2000, pages 39-46, XP005076104 ISSN: 1084-8592
- TRICARICO CARMELA ET AL: "Quantitative real-time reverse transcription polymerase chain reaction: Normalization to rRNA or single housekeeping genes is inappropriate for human tissue biopsies." ANALYTICAL BIOCHEMISTRY, vol. 309, no. 2, 15 October 2002 (2002-10-15), pages 293-300, XP002472530 ISSN: 0003-2697
- DE JONGE HENDRIK J M ET AL: "Evidence based selection of housekeeping genes." PLOS ONE 2007, vol. 2, no. 9, September 2007 (2007-09), page e898, XP002472572 ISSN: 1932-6203

## Description

The present invention relates to a method for the prognosis and prediction of breast cancer.

The metastatic potential of primary tumors is the chief prognostic determinant of this malignant disease. Therefore, predicting the risk of a patient developing a metastasis is an important factor in predicting the outcome of disease and choosing an appropriate treatment.

As an example, breast cancer is the leading cause of death in women between the ages of 35-55. Worldwide, there are over 3 million women living with breast cancer. OECD (Organization for Economic Cooperation & Development) estimates on a worldwide basis 500,000 new cases of breast cancer are diagnosed each year. One out of ten women will face the diagnosis breast cancer at some point during her lifetime. Breast cancer is the abnormal growth of cells that line the breast tissue ducts and lobules and is classified by whether the cancer started in the ducts or the lobules and whether the cells have invaded (grown or spread) through the duct or lobule, and by the way the cells look under the microscope (tissue histology). Other forms of breast cancer exist, making this a very heterogeneous disease. It is not unusual for a single breast tumor to have a mixture of invasive and in situ cancer. According to today's therapy guidelines and current medical practice, the selection of a specific therapeutic intervention is mainly based on histology, grading, staging, hormonal status, and RNA/DNA amplification (such as Her2/neu) of the patient. Many aspects of a patient's specific type of tumor are currently not assessed - preventing true patient-tailored treatment. Another dilemma of today's breast cancer therapeutic regimens is the practice of significant over-treatment of patients; it is well known from past clinical trials that 70% of breast cancer patients with early stage disease do not need any treatment beyond surgery which may be followed by radiotherapy. While about 90% of all early stage cancer patients receive chemotherapy exposing them to significant treatment side effects, approximately 30% of these relapse. These types of problems are common to other forms of cancer as well. As such, there is a significant medical need to develop diagnostic assays that identify low risk patients for directed therapy. For patients with medium or high risk assessment, there is a need to pinpoint therapeutic regimens tailored to the specific cancer to assure optimal success. Breast Cancer metastasis and disease-free survival prediction is a challenge for all pathologists and treating oncologists. A test that can predict such features has a high medical and diagnostic need.

About 80% of all breast cancers diagnosed in the US and Europe are node-negative. What is needed, is a test which can assess the risk of development of metastasis.

Technologies such as quantitative PCR, microarray analysis, and others allow the analysis of genome-wide expression pattern which provides new insights into gene regulation and is also a useful diagnostic tool, because it allows the analysis of pathologic conditions at the level of gene expression. Quantitative reverse transcriptase PCR is currently the accepted standard for quantifying gene expression. It has the advantage of being a very sensitive method allowing the detection of even minute amounts of mRNA.

Commonly, the expression level of a gene of interest is measured relative to the expression level of an internal control gene. As internal control, it is known to use so called housekeeping genes.

Housekeeping genes are vital to the metabolism of viable cells and are therefore constantly expressed. Many housekeeping genes encode enzymes or structural RNAs, such as ribosomal RNAs, which perform essential metabolic functions (hence the name "housekeeping") and are therefore constantly expressed. Table 1 lists some exemplary housekeeping genes. The prerequisite of a suitable housekeeping gene is that it should, of course, be adequately expressed in the tissue of interest, but most importantly, that it shows minimal variability in expression between samples and under the experimental conditions used. A selection of housekeeping genes is, for example shown in Vandesompele et al., Genome Biol. 2002 Jun 18;3(7):RESEARCH0034. Epub 2002 Jun 18.

Rondinelli R.H. et al show an increased GAPDH gene expression in late pathological stage human prostate cancer, but do not show any prognostic value for determining GAPDH gene expression in relation to RPL37A (Rondinelli R.H. et al in Prostate Cancer and Prostatic Diseases, Stockton Press, Basingstoke, GB, vol. 1, No. 2, December 1997, pages 66-72, XP009024855, ISSN: 1365-7852).

**Table 1: Exemplary housekeeping genes (from: Vandesompele et al.)**

| Symbol | Accession Number, Gene Title | | Function |
|---|---|---|---|
| *ACTB* | NM_001101 | Beta actin | Cytoskeletal structural protein |
| *B2M* | NM_004048 | Beta-2-microglobulin | Beta-chain of major histocompatibility complex class I molecules |
| *GAPD* | NM_002046 | Glyceraldehyde-3-phosphate dehydrogenase | Oxidoreductase in glycolysis and gluconeogenesis |
| *HMBS* | NM_000190 | Hydroxymethyl-bilane synthase | Heme synthesis, porphyrin metabolism |
| *HPRT1* | NM_000194 | Hypoxanthine phosphoribosyl-transferase 1 | Purine synthesis in salvage pathway |
| *RPL13A* | NM_012423 | Ribosomal protein L13a | Structural component of the large 60S ribosomal subunit |
| *SDHA* | NM_004168 | Succinate dehydrogenase complex, subunit A | Electron transporter in the TCA cycle and respiratory chain |
| *TBP* | NM_003194 | TATA box binding protein | General RNA polymerase II transcription factor |
| *UBC* | M26880 | Ubiquitin C | Protein degradation |
| *YWHAZ* | NM_003406 | Tyrosine 3-monooxygenase/ tryptophan 5-monooxygenase binding to phosphorylated activation protein, zeta polypeptide | Signal transduction |

In qPCR studies, it is common to measure up to six or more housekeeping genes in parallel to the gene of interest. A CT value (cycle threshold of exponential growth in qPCR) is used as quantifiable signal, and gene expression is determined using the mean of expression levels of the housekeeping genes (normalization standard) and the expression levels of a gene of interest. This type of normalization or control is required because several variables need to be controlled for gene expression analysis, e.g. the amount of starting material, the quality of RNA, differences between tissues or cells in overall transcriptional activity and so on. The level of gene expression of a gene of interest (GOI) may then be expressed relative to the expression of the control (i.e. housekeeping gene), e.g. as delta CT = CT[GOI] - CT[control].

Curing breast cancer patients is still a challenge for the treating oncologist as the diagnosis relies in most cases on clinical and pathological data like age, menopausal status, hormonal status, grading, and general constitution of the patient and some molecular markers like Her2/neu, p53, and some others. Unfortunately, until recently, there was no test in the market for prognosis or therapy prediction that comes up with a more elaborated recommendation for the treating oncologist whether and how to treat patients. Two assay systems are available for prognosis, Genomic Health's OncotypeDX and Agendia's Mammaprint assay. In 2007, the company Agendia got FDA approval for their Mammaprint microarray assay that can predict with the help of 70 informative genes and a bundle of housekeeping genes the prognosis of breast cancer patients from fresh tissue. The Genomic Health assay works with formalin-fixed and paraffin-embedded tumor tissue samples and uses 21 genes for the prognosis, presented as a risk score.

Both these assays use a high number of different markers to arrive at a result. What is needed, is a simple and robust assay for prediction and/or prognosis of cancer.

### Objective of the invention

It is an objective of the invention to provide a method for the prediction and/or prognosis of breast cancer relying on a limited number of markers.

### Definitions

The term "neoplastic disease", "neoplastic region", or "neoplastic tissue" refers to a tumorous tissue including carcinoma (e.g. carcinoma in situ, invasive carcinoma, metastasic carcinoma) and pre-malignant conditions, neomorphic changes independent of their histological origin, cancer, or cancerous disease.

The term "cancer" is not limited to any stage, grade, histomorphological feature, aggressivity, invasiveness (e.g. ductal carcinoma in situ (DCIS) or lobular carcinoma in situ (LCIS), or invasive carcinoma) or degree of malignancy of an affected tissue or cell aggregation. In particular, solid tumors, malignant lymphoma and all other types of cancerous tissue, malignancy and transformations associated therewith, lung cancer, ovarian cancer, cervix cancer, stomach cancer, pancreas cancer, prostate cancer, head and neck cancer, renal cell cancer, colon cancer or breast cancer are included. The terms "neoplastic lesion" or "neoplastic disease" or "neoplasm" or "cancer" are not limited to any tissue or cell type. They also include primary, secondary, or metastatic lesions of cancer patients, and also shall comprise lymph nodes affected by cancer cells or minimal residual disease cells either locally deposited or freely floating throughout the patient's body.

The term "predicting an outcome" of a disease, as used herein, is meant to include both a prediction of an outcome of a patient undergoing a given therapy and a prognosis of a patient who is not treated. The term "predicting an outcome" may, in particular, relate to the risk of a patient suffering an event, such as metastasis or death, preferably within a given time frame.

The term "prediction", as used herein, relates to an individual assessment of the malignancy of a tumor, or to the expected survival rate (DFS, disease free survival) of a patient, if the tumor is treated with a given therapy. In contrast thereto, the term "prognosis" relates to an individual assesment of the malignancy of a tumor, or to the expected survival rate (DFS, disease free survival) of a patient, if the tumor remains untreated.

A "discriminant function" is a function of a set of variables used to classify an object or event. A discriminant function thus allows classification of a patient, sample or event into a category or a plurality of categories according to data or parameters available from said patient, sample or event. Such classification is a standard instrument of statistical analysis well known to the skilled person. E.g. a patient may be classified as "high risk" or "low risk", "high probability of metastasis" or "low probability of metastasis", "in need of treatment" or "not in need of treatment" according to data obtained from said patient, sample or event. Classification is not limited to "high vs. low", but may be performed into a plurality categories, grading or the like. Examples for discriminant functions which allow a classification include, but are not limited to discriminant functions defined by support vector machines (SVM), k-nearest neighbors (kNN), (naive) Bayes models, or piecewise defined functions such as, for example, in subgroup discovery, in decision trees, in logical analysis of data (LAD) an the like.

In the context of the present invention, a "biological sample" is a sample which is derived from or has been in contact with a biological organism. Examples for biological samples are: cells, tissue, body fluids, biopsy specimens, blood, urine, saliva, sputum, plasma, serum, cell culture supernatant, and others.

A "biological molecule" within the meaning of the present invention is a molecule generated or produced by a biological organism or indirectly derived from a molecule generated by a biological organism, such as: nucleic acids, protein, polypeptide, peptide, DNA, mRNA, cDNA, and so on.

A "probe" is a molecule or substance capable of specifically binding or interacting with a specific biological molecule. The term "primer", "primer pair" or "probe", shall have ordinary meaning of these terms which is known to the person skilled in the art of molecular biology. In a preferred embodiment of the invention, "primer", "primer pair" and "probes" refer to oligonucleotide or polynucleotide molecules with a sequence identical to, complementary too, homologues of, or homologous to regions of the target molecule or target sequence which is to be detected or quantified, such that the primer, primer pair or probe can specifically bind to the target molecule, e.g. target nucleic acid, RNA, DNA, cDNA, gene, transcript, peptide, polypeptide, or protein to be detected or quantified. As understood herein, a primer may in itself function as a probe. A "probe" as understood herein may also comprise e.g. a combination of primer pair and internal labeled probe, as is common in many commercially available qPCR methods.

A "gene" is a set of segments of nucleic acid that contains the information necessary to produce a functional RNA product in a controlled manner. A "gene product" is a biological molecule produced through transcription or expression of a gene, e.g. an mRNA or the translated protein.

An "mRNA" is the transcribed product of a gene and shall have the ordinary meaning understood by a person skilled in the art. A "molecule derived from an mRNA" is a molecule which is chemically or enzymatically obtained from an mRNA template, such as cDNA.

The term "specifically binding" within the context of the present invention means a specific interaction between a probe and a biological molecule leading to a binding complex of probe and biological molecule, such as DNA-DNA binding, RNA-DNA binding, RNA-RNA binding, DNA-protein binding, protein-protein binding, RNA-protein binding, antibody-antigen binding, and so on.

The term "expression level" refers to a determined level of gene expression. This may be a determined level of gene expression compared to a reference gene (e.g. a housekeeping gene) or to a computed average expression value (e.g. in DNA chip analysis) or to another informative gene without the use of a reference sample. The expression level of a gene may be measured directly, e.g. by obtaining a signal wherein the signal strength is correlated to the amount of mRNA transcripts of that gene or it may be obtained indirectly at a protein level, e.g. by immunohistochemistry, CISH, ELISA or RIA methods. The expression level may also be obtained by way of a competitive reaction to a reference sample.

The term "complementary" or "sufficiently complementary" means a degree of complementarity which is - under given assay conditions - sufficient to allow the formation of a binding complex of a primer or probe to a target molecule. Assay conditions which have an influence of binding of probe to target include temperature, solution conditions, such as composition, pH, ion concentrations, etc. as is known to the skilled person.

The term "hybridization-based method", as used herein, refers to methods imparting a process of combining complementary, single-stranded nucleic acids or nucleotide analogues into a single double stranded molecule. Nucleotides or nucleotide analogues will bind to their complement under normal conditions, so two perfectly complementary strands will bind to each other readily. In bioanalytics, very often labeled, single stranded probes are used in order to find complementary target sequences. If such sequences exist in the sample, the probes will hybridize to said sequences which can then be detected due to the label. Other hybridization-based methods comprise microarray and/or biochip methods. Therein, probes are immobilized on a solid phase, which is then exposed to a sample. If complementary nucleic acids exist in the sample, these will hybridize to the probes and can thus be detected. Hybridization is dependent on target and probe (e.g. length of matching sequence, GC content) and hybridization conditions (temperature, solvent, pH, ion concentrations, presence of denaturing agents, etc.). A "hybridizing counterpart" of a nucleic acid is understood to mean a probe or capture sequence which under normal conditions hybridizes to said nucleic acid and forms a binding complex with said nucleic acid. Normal conditions refers to temperature and solvent conditions and are understood to mean conditions under which a probe can hybridize to allelic variants of a nucleic acid but does not unspecifically bind to unrelated genes. These conditions are known to the skilled person and are e.g. described in "Molecular Cloning. A laboratory manual", Cold Spring Harbour Laboratory Press, 2nd Edition, 1989. Normal conditions would be e.g. hybridization at 6 x Sodium Chloride/sodium citrate buffer (SSC) at about 45°C, followed by washing or rinsing with 2 x SSC at about 50°C, or e.g. conditions used in standard PCR protocols, such as annealing temperature of 40 to 60°C in standard PCR reaction mix or buffer.

The term "array" refers to an arrangement of addressable locations on a device, e.g. a chip device. The number of locations can range from several to at least hundreds or thousands. Each location represents an independent reaction site. Arrays include, but are not limited to nucleic acid arrays, protein arrays and antibody-arrays. A "nucleic acid array" refers to an array containing nucleic acid probes, such as oligonucleotides, polynucleotides or larger portions of genes. The nucleic acid on the array is preferably single stranded. A "microarray" refers to a biochip or biological chip, i.e. an array of regions having a density of discrete regions with immobilized probes of at least about 100/cm²

A "PCR-based method" refers to methods comprising a polymerase chain reaction (PCR). This is a method of exponentially amplifying nucleic acids, e.g. DNA or RNA, by enzymatic replication in vitro using one, two or more primers. For RNA amplification, a reverse transcription may be used as a first step. PCR-based methods comprise kinetic or quantitative PCR (qPCR) which is particularly suited for the analysis of expression levels.

The term "determining a protein level" refers to any method suitable for quantifying the amount, amount relative to a standard or concentration of a given protein in a sample. Commonly used methods to determine the amount of a given protein are e.g. immunohistochemistry, CISH, ELISA or RIA methods. etc.

The term "reacting" a probe with a biological molecule to form a binding complex herein means bringing probe and biologically molecule into contact, for example, in liquid solution, for a time period and under conditions sufficient to form a binding complex.

The term "label" within the context of the present invention refers to any means which can yield or generate or lead to a detectable signal when a probe specifically binds a biological molecule to form a binding complex. This can be a label in the traditional sense, such as enzymatic label, fluorophore, chromophore, dye, radioactive label, luminescent label, gold label, and others. In a more general sense the term "label" herein is meant to encompass any means capable of detecting a binding complex and yielding a detectable signal, which can be detected, e.g. by sensors with optical detection, electrical detection, chemical detection, gravimetric detection (i.e. detecting a change in mass), and others. Further examples for labels specifically include labels commonly used in qPCR methods, such as the commonly used dyes FAM, VIC, TET, HEX, JOE, Texas Red, Yakima Yellow, quenchers like TAMRA, minor groove binder, dark quencher, and others or probe indirect staining of PCR products by for example SYBR Green. Readout can be performed on hybridization platforms, like Affymetrix, Agilent, Illumina, Planar Wave Guides, Luminex, microarray devices with optical, magnetic, electrochemical, gravimetric detection systems, and others. A label can be directly attached to a probe or indirectly bound to a probe, e.g. by secondary antibody, by biotin-streptavidin interaction or the like.

The term "combined detectable signal" within the meaning of the present invention means a signal, which results, when at least two different biological molecules form a binding complex with their respective probes and one common label yields a detectable signal for either binding event.

### Summary of the invention

The invention is predicated on the discovery that a simple copy number ratio or gene expression level of two genes, RPL37A and GAPDH, already exhibits a powerful predictive power for assessing the outcome of a neoplastic or cancerous disease and is defined by claims 1 and 9.

Further disclosed herein is a method for predicting an outcome of a patient suffering from or at risk of developing a neoplastic disease, said method comprising the steps of:
a) obtaining a biological sample from said patient;
b) quantifiably determining the gene expression level of a first housekeeping gene selected from one of GAPDH and RPL37A, and quantifiably determining the gene expression level at least one further housekeeping gene different from said first housekeeping gene, wherein the expression level of said one of GAPDH and RPL37A is determined in relation to the expression level of said at least one further housekeeping gene;
c) arriving at a prediction by means of a mathematical discriminant function of the expression level of one of GAPDH and RPL37A in relation to the expression level of said one further housekeeping gene.

According to a further aspect disclosed herein, the expression level of GAPDH is determined in relation to the expression level of a plurality of further housekeeping genes.

According to a further aspect disclosed herein, the expression level of a plurality of further housekeeping genes is determined by forming a mathematical average of the expression level of each of the plurality of further housekeeping genes.

According to a further aspect disclosed herein, the expression level of a plurality of further housekeeping genes is determined by using a mixture of probes for each of the plurality of further housekeeping genes wherein the probes yield a combined detectable signal indicative of the expression level of the plurality of further housekeeping genes.

According to a further aspect disclosed herein the at least one further housekeeping gene is selected from the group consisting of GAPDH, RPL37A, ACTG1, PPIA, CALM2, and OAZ1.

According to preferred aspect disclosed herein said first housekeeping gene is GAPDH.

According to a further preferred aspect disclosed herein the expression level of GAPDH is determined in relation to the expression level of RPL37A.

According to a further aspect of the invention the mathematical discriminate function comprises classifying the sample into one of at least two categories.

According to a further aspect of the invention the mathematical discriminate function is a comparison with one, two or more predetermined threshold values.

According to a further aspect of the invention, the expression level is determined by
a) a hybridization-based method;
b) a PCR-based method;
c) determining the protein level,
d) a method based on the electrochemical detection of particular molecules, and/or by
e) an array-based method.

According to a further aspect of the invention, the expression level of GAPDH and RPL37A is determined with RT-PCR (reverse transcriptase polymerase chain reaction) of the GAPDH mRNA or RPL37A mRNA.

According to a further aspect of the invention, the expression level is determined in formalin and/or paraffin fixed tissue samples.

According to a further aspect of the invention, after lysis, the samples are treated with silica-coated magnetic particles and a chaotropic salt, in order to purify the nucleic acids contained in said sample for further determination.

Further disclosed herein is a kit for performing a method according to any of the preceding claims, comprising:
a) a first probe useful for determining a gene expression level of one of GAPDH and RPL37A;
b) a first label specific for said first probe;
c) a second probe different from said first probe useful for determining a gene expression level of at least one further housekeeping gene; and
d) a second label specific for said second probe.

The second label is distinguishable from the first label. A label "specific for a probe" means a label which will associate specifically with the one probe and not the other probe. This can be achieved by direct attachment of the label to the probe or through a specific interaction of label and probe, e.g. a sequence specific interaction, an antibody-antigen interaction, a ligand-komplex interaction or the like.

According to a further aspect disclosed herein, the first probe is a nucleic acid capable of forming a binding complex with an mRNA or a molecule derived from an mRNA of GAPDH or RPL37A.

According to a further aspect disclosed herein, the second probe is a nucleic acid capable of forming a binding complex with an mRNA or a molecule derived from an mRNA of the at least one further housekeeping gene.

According to a further aspect disclosed herein, the first probe is labelled with a first label and the second probe is labelled with a second label distinguishable from the first label.

According to a further aspect disclosed herein, the first probe is a nucleic acid selected from the group consisting of SEQ ID NO: 1, 2, 7, 8, 13, 14, 19, 20, and fractions, variants, homologues, derivatives, fragments, complements, hybridizing counterparts, or molecules sharing a sequence identity of at least 70%, preferably 95%.

According to a further aspect disclosed herein, for the kit, the at least one further housekeeping gene is selected from the group consisting of GAPDH, RPL37A, ACTG1, PPIA, CALM2, and OAZ1.

According to a further aspect disclosed herein, the second probe is a nucleic acid selected from the group consisting of SEQ ID NO: 1 to 24 and fractions, variants, homologues, derivatives, fragments, complements, hybridizing counterparts, or molecules sharing a sequence identity of at least 70%, preferably 95%.

The invention further relates to the use of a nucleic acid for performing the method according to the invention, said nucleic acid being selected from the group consisting of
a) SEQ ID NO: 1, 2, 7, 8, 13, 14, 19, 20 and fractions, fragments, complements, hybridizing counterparts thereof, or molecules sharing a sequence identity of at least 70%, preferably 95%;
b) a hybridizing counterpart of a nucleic acid according to SEQ ID NO: 25 or SEQ ID NO: 26;
c) a nucleic acid complementary to a fragment of at least 15 contiguous nucleotides of SEQ ID NO: 25 or SEQ ID NO: 26; and
d) a nucleic acid comprising a sequence of at least 15 contiguous nucleotides of SEQ ID NO: 25 or SEQ ID NO: 26.

The method of the invention is useful for predicting an outcome of a patient suffering from breast cancer

The method of the invention can e.g. be used to stratify diagnosed lymph node-negative breast cancer patients into low, (medium) or high risk groups. The stratification will provide the treating oncologist with the likelihood that the tested patient will suffer from cancer recurrence or develop a metastasis in the absence of therapy. The oncologist can utilize the results of this test to make decisions on therapeutic regimens.

According to an aspect of the present invention the expression level may be determined by one or more of the following methods:
a) a hybridization-based method;
b) a PCR-based method;
c) determining the protein level;
   and
d) an array-based method.

Gene expression may be determined by quantitative PCR. This method can be run on any quantitative PCR system. Commercially available systems may be used, for example, "Real Time PCR System" of Applied Biosystems, LightCycler® from Roche, iCycler® from BioRAd, Mx from Stratagene, and others.

According to yet another different aspect of the present invention the expression of genes can be analyzed at a protein-level. For this type of assay an ELISA or RIA format may be used.

The method of the present invention can be performed as multiplex assay in single reaction vessel type formats (e.g. an Eppendorf cup).

The method of the present invention can readily be adapted to microtiter plate formats, e.g. by precoating wells with the first group of probes using a first label and possibly a second group of probes using a second label or a further probe for an individual gene of interest using a further label.

Advantages over existing methods are the very low number of genes needed for prognosis and the use of standard primer /probe sets.

The invention relies on the measurement of the expression level of GAPDH and RPL37A in breast cancer tissue samples, The fact that the gene expression of GAPDH relative to RPL37A and further housekeeping genes is of high prognostic value was surprising, but reproducible and statistically significant results confirming this fact across unrelated cohorts were obtained. Therefore, according to the invention, the expression level of GAPDH is determined in relation to the expression level of RPL37A.

It is surprising that while GAPDH and RPL37A are frequently used as housekeeping gene, nobody has actually discovered the prognostic value of these markers in relation to other housekeeping genes. Previously, GAPDH and RPL37A have also been widely used as housekeeping gene for internal controls. The inventors of the present invention have determined that GAPDH, in particular, should not be chosen as control gene in gene expression analysis, since it is regulated in hypoxia, i.e. when the cell does not receive a sufficient amount of oxygen. It is thought that this may in fact be the case for fast-growing tumors where growth is limited by the insufficient vascularization, so tumor cells would be partly or to a greater extent in a state of hypoxia, and consequently this would be represented on the molecular level among other genes by the expression of GAPDH. In the copy number ratio (delta of the CT values), GAPDH is compared to a further housekeeping gene with a constant expression in the investigated disease setting, which is used to determine up- or downregulation of GAPDH.

Glyceraldehyde 3-phosphate dehydrogenase (abbreviated as GAPD, GAPDH, or less commonly as G3PDH) is an enzyme involved in the catalysis of glycolysis and thus serves to break down glucose for energy and carbon molecules. In addition to this long established metabolic function, GAPDH has recently been implicated in several non-metabolic processes, including transcription activation, initiation of apoptosis, and ER to Golgi vesicle shuttling.

In 2003 Zheng et al. showed that GAPDH can itself activate transcription. The OCA-S transcriptional coactivator complex contains GAPDH and lactate dehydrogenase, two proteins previously only thought to be involved in metabolism. GAPDH moves between the cytosol and the nucleus and may thus link the metabolic state to gene transcription (Zheng L, Roeder RG, Luo Y (2003). "S phase activation of the histone H2B promoter by OCA-S, a coactivator complex that contains GAPDH as a key component". Cell 114 (2): 255-66). Further, Hara et al. discovered in 2005 that GAPDH also regulates apoptosis. This is not a third function, but can be seen as an activity mediated by GAPDH binding to DNA like in transcription activation, discussed above (Hara MR, Agrawal N, Kim SF, et al (2005). "S-nitrosylated GAPDH initiates apoptotic cell death by nuclear translocation following Siahl binding". Nat. Cell Biol. 7 (7): 665-74). The mRNA sequence of GAPDH is given as SEQ ID NO:25 in the attached sequence listing.

Database links for GAPDH are:
Entrez: 2597
OMIM: 138400
RefSeq: NM_002046
UniProt: P04406

The ribosomal Protein L37a (RPL37A) is part of the ribosomal complex. Ribosomes, the organelles that catalyze protein synthesis, consist of a small 40S subunit and a large 60S subunit. Together these subunits are composed of 4 RNA species and approximately 80 structurally distinct proteins. The RPL37A gene encodes a ribosomal protein that is a component of the 60S subunit. The protein belongs to the L37AE family of ribosomal proteins. It is located in the cytoplasm. The protein contains a C₄-type zinc finger-like domain. (Hoof T et al, "Primary sequence of the human ribosomal protein L37a" Nucleic Acids Res. 1992 Oct 25;20(20) :5475). The mRNA sequence of RPL37A is given as SEQ ID NO:26 in the attached sequence listing.

Database links for RPL37A are:
Entrez: 6168
RefSeq: NM_000998
UniProt: P61513

The invention is now described in more detail in connection with the Figures, in which:
Figure 1 shows a Kaplan Meier plot showing metastasis-free survival time of node-negative non-metastasized patients divided into high and low risk of metastasis according to the method of the invention. The threshold is the median of all Delta CT values (as indicated in the legend).
Figure 2 shows a Kaplan Meier plot showing metastasis-free survival time of node-negative non-metastasized patients divided into high, intermediate, and low risk of metastasis according to the method of the invention. The thresholds are the first and second tercile of the Delta CT values (as indicated in the legend).
Figure 3 shows a Kaplan Meier plot showing overall survival time of metastasized patients divided into high, and low risk of death according to the method of the invention. The threshold is the median of all Delta CT values (as indicated in the legend).
Figure 4 shows a Kaplan Meier plot showing overall survival time of metastasized patients divided into high, intermediate, and low risk of death according to the method of the invention. The thresholds are the first and second tercile of the Delta CT values (as indicated in the legend).

Technically, the assay used in these examples relies on two core technologies: 1.) Isolation of total RNA from tumor tissue and 2.) quantification of mRNA by e.g. kinetic RT-PCR.

The assay results can be linked together by a mathematical algorithm computing the likely risk of getting metastasis as low, (intermediate) or high, which may be implemented in a software.

In case of RNA analysis to determine an expression level, RNA may be isolated by any known suitable method, e.g. using commercially available RNA isolation kits.

According to a preferred embodiment of the present invention, after lysis the samples are treated with silica-coated magnetic particles and a chaotropic salt, in order to purify nucleic acids contained in the sample for further analysis. This method of RNA isolation has been shown to yield satisfactory results even when RNA is extracted from fixed tissue samples. This method which allows successful purification of mRNA out of fixed tissue samples is disclosed in WO 030058649, WO 2006136314A1 and DE10201084A1, the content of which is incorporated herein by reference.

This RNA extraction method comprises the use of magnetic particles coated with silica (silicon dioxide, SiO₂). These highly pure magnetic particles coated with silica are used for isolating nucleic acids, including DNA and RNA from cell and tissue samples, the particles, the particles may be retrieved from a sample matrix or sample solution by use of a magnetic field. These particles are particularly well-suited for the automatic purification of nucleic acids, mostly from biological samples for the purpose of detecting nucleic acids.

The selective binding of nucleic acids to the surface of these particles is due to the affinity of negatively charged nucleic acids to silica containing media in the presence of chaotropic salt like guanidine isothiocyanate. The binding properties are for example described in EP 819696.

This approach is particularly useful for the purification of mRNA from formalin and/or paraffin fixed tissue samples. In contrast to most other approaches which may result in small fragments that are not well-suited for later gene expression analysis by PCR and/or hybridization technologies, this approach creates mRNA fragments which are large enough to allow such analysis.

This approach allows a highly-specific determination of gene expression levels with one of the above-mentioned methods, in particular hybridization-based methods, PCR-based methods and/or array-based methods even in formalin and/or paraffin-fixed tissue samples and is therefore highly efficient in the context of the present invention, because it allows the use of fixed tissue samples which often are readily available in tissue banks and connected to clinical data bases, e.g. for follow-up studies to allow retrospective analysis.

Measurement of the expression level can be performed on the mRNA level by any suitable method, e.g. qPCR or gene expression array platforms; including, but not limited to, commercially available platforms, such as TaqMan^{®}, Lightcycler^{®}, Affymetrix, Illumina, Luminex, planar wave guide, electrochemical microarray chips, microarray chips with optical, magnetic, electrochemical or gravimetric detection systems and others or on a protein level by immunochemical techniques such as ELISA.

The term "planar waveguide" relates to methods, wherein the presence or amount of a target molecule is determined by using a planar wave guide detector which emits an evanescent field in order to detect the binding of a labeled target molecule, such as e.g. described in WO200113096-A1 the content of which is incorporated by reference herein.

The term "optical detection" relates to methods which detect the presence or amount of a target molecule through a change in optical properties, e.g. fluorescence, absorption, reflectance, chemiluminescence, as is well known in the art.

The term "magnetic detection" relates to methods which detect the presence or amount of a target molecule or label through a change in magnetic properties, e.g. through the use of XMR sensors, GMR sensors or the like.

The term "electrochemical detection" relates to methods which make use of an electrode system to which molecules, particularly biomolecules like proteins, nucleic acids, antigens, antibodies and the like, bind under creation of a detectable signal. Such methods are for example disclosed in WO0242759, WO200241992 and WO2002097413, the content of which is incorporated by reference herein. These detectors comprise a substrate with a planar surface and electrical detectors which may adopt, for example, the shape of interdigital electrodes or a two dimensional electrode array. These electrodes carry probe molecules, e.g. nucleic acid probes, capable of binding specifically to target molecules, e.g. target nucleic acid molecules and allowing a subsequent electrochemical detection of bound target nucleic acids.

The term "gravimetric detection" relates to methods which make use of a system that is able to detect changes in mass which occur e.g. when a probe binds its target.

In a representative example, quantitative reverse transcriptase PCR was performed according to the following protocol:
Primer/Probe Mix:

| | | |
|---|---|---|
| 50 µl | 100 µM | Stock Solution Forward Primer |
| 50 µl | 100 µM | Stock Solution Reverse Primer |
| 25 µl | 100 µM | Stock Solution Taq Man Probe bring to 1000 µl with water |
| 10 µl | Primer/Probe Mix (1:10) are lyophilized, 2.5 h RT | |

RT-PCR Assay set-up for 1 well:

| | |
|---|---|
| 3.1 µl | Water |
| 5.2 µl | RT qPCR MasterMix (Invitrogen) & Rox |
| 0.5 µl | MgSO4 (to 5.5 mM final concentration) |
| 1 µl | Primer/Probe Mix dried |
| 0.2 µl | RT/Taq Mx (-RT: 0.08 µL Taq) |
| 1 µl | RNA (1:2) |

Thermal Profile:

| RT step | | |
|---|---|---|
| 50 °C | | 30 Min * |
| 8 °C | ca. | 20 Min * |
| 95 °C | | 2 Min |

| PCR cycles (repeated for 40 cycles) | |
|---|---|
| 95 °C | 15 Sec. |
| 60 °C | 30 Sec. |

Table 2 shows probe sequences used in qPCR (termed qPCR-probe), tables 3 and 4 show the respective forward and reverse primer sequences and table 5 shows the sequences of the obtained amplicons/PCR products. Sequences are identified by respective SEQ ID NOs. RPL37A, ACTG1, PPIA, CALM2, and OAZ1 are examples of genes which can be used as further housekeeping gene in the method of the invention. With the exception of GAPDH, the genes disclosed in table 1 can also be used as further housekeeping gene in the method of the invention.

**Table 2: qPCR-Probe Sequences**

| **Gene** | **RefSeq or ID** | **Probe** | **SEQ ID NO:** | **FAM 5' Sequence 3' TAMRA** |
|---|---|---|---|---|
| GAPD | NM_02046 | R15 | 1 | |
| RPL37A | NM_000998 | R16 | 2 | TGGCTGGCGGTGCCTGGA |
| ACTG1 | NM_001614 | R113 | 3 | AGGCCCCCCTGAACCCCAAG |
| PPIA | NM_021130 | R115 | 4 | TGGTTGGATGGCAAGCATGTGGTG |
| CALM2 | NM_001743 | R117 | 5 | TCGCGTCTCGGAAACCGGTAGC |
| OAZ1 | NM_004152 | BC268 | 6 | TGCTTCCACAAGAACCGCGAGGA |

**Table3: Forward Primer Sequences**

| **Gene** | **5' Primer** | **SEQ ID NO:** | **5' Sequence 3'** |
|---|---|---|---|
| GAPD | R15-for | 7 | GCC AGC CGA GCC ACA TC |
| RPL37A | R16-for | 8 | TGTGGTTCCTGCATGAAGACA |
| ACTG1 | R113-for | 9 | CTGGCACCACACCTTCTACAAC |
| PPIA | R115-for | 10 | TTTCATCTGCACTGCCAAGACT |
| CALM2 | R117-for | 11 | GAGCGAGCTGAGTGGTTGTG |
| OAZ1 | BC268-for | 12 | CGAGCCGACCATGTCTTCAT |

**Table 4: Reverse Primer Sequences**

| **Gene** | **3' Primer** | **SEQ ID NO:** | **5' Seqence 3'** |
|---|---|---|---|
| GAPD | R15rev | 13 | CCA GGC GCC CAA TAC G |
| RPL37A | R16rev | 14 | GTGACAGCGGAAGTGGTATTGTAC |
| ACTG1 | R113-rev | 15 | CAAACATAATCTGAGTCATCTTCTCTCTGT |
| PPIA | R115-rev | 16 | TATTCATGCCTTCTTTCACTTTGC |
| CALM2 | R117-rev | 17 | AGTCAGTTGGTCAGCCATGCT |
| OAZ1 | BC268-rev | 18 | AAGCCCAAAAAGCTGAAGGTT |

**Table 5: Gene amplicons**

| **Gene** | **SEQ ID NO:** | **Amplicon** |
|---|---|---|
| GAPD | 19 | |
| RPL37A | 20 | |
| ACTG1 | 21 | |
| PPIA | 22 | |
| CALM2 | 23 | |
| OAZ1 | 24 | |

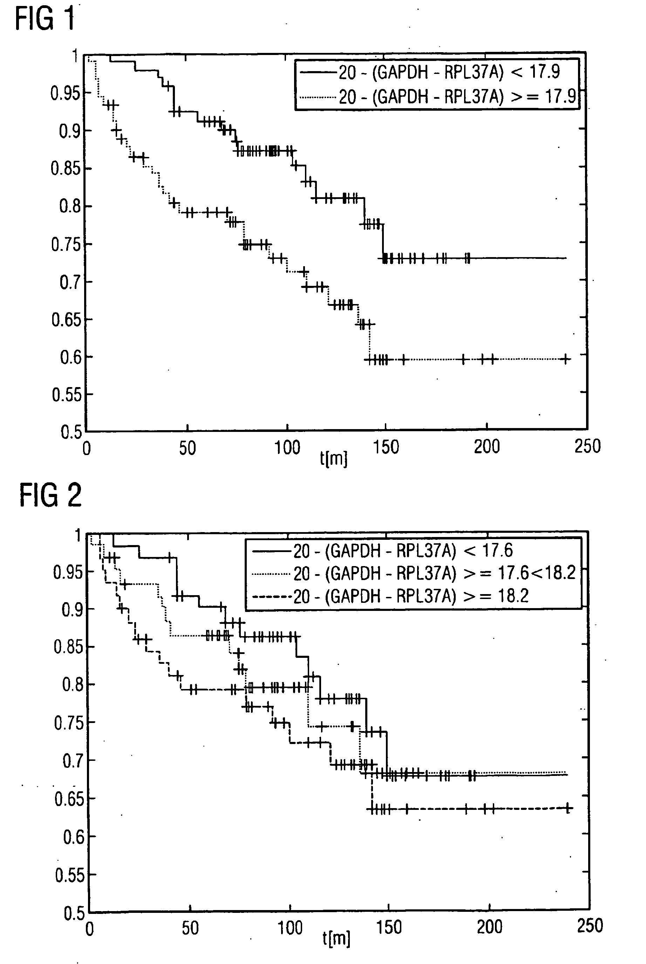

In the examples of Figures 1 to 4, the ratio of GAPDH as said first housekeeping gene and RPL37A as said at least one further house keeping gene was chosen for analysis. The mRNA concentration of GAPDH and RPL37A was determined in 172 node-negative, untreated breast-cancer tumor tissue samples and a highly significant, stable relation between time to metastasis and the delta-CT value of GAPDH and RPL37A was found, which is depicted in Figs. 1 and 2.

Figure 1 depicts a Kaplan-Meier plot showing metastasis free survival time of node-negative non-metastasized patients divided into high and low risk of metastasis according to the method of the invention. X-axis shows metastasis free survival time in months, Y-axis shows percentage of patients with metastasis free survival. Patients were discretized into two bins "GAPDH high" and "GAPDH low" with separation at the median. Relative GAPDH level was expressed "20 - delta CT", wherein delta CT = CT[GAPDH] - CT [RPL37A]. It can be clearly seen that "GAPDH low" patients have a significantly better disease outcome than "GAPDH high" patients (p=1.1%).

Figure 2 shows a Kaplan-Meier plot showing metastasis free survival time of node-negative non-metastasized patients divided into high, intermediate, and low risk of metastasis according to the method of the invention. X-axis shows metastasis free survival time in months, Y-axis shows percentage of patients with metastasis free survival. Patients were discretized into three bins "GAPDH high", "GAPDH intermediate" and "GAPDH low" with separation at the terciles. Relative GAPDH level was expressed as "20 - delta CT", wherein delta CT = CT [GAPDH] - CT [RPL37A].

The observation was reproduced in a very different breast cancer cohort, where all patients were already in an advanced, metastasized stage of the disease, so the target quantity here was to look at overall survival of the patients. Again, it was found that the CT difference in GAPDH and RPL37A was a significant, strong predictor of disease outcome. This cohort comprised 299 patients.

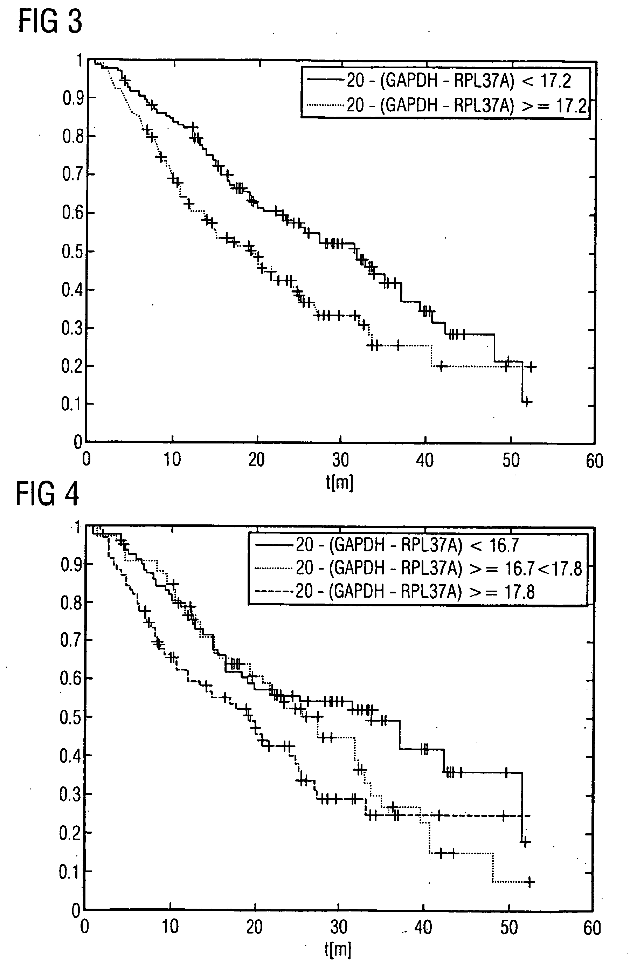

Figure 3 depicts a Kaplan-Meier plot showing metastasis free survival time of advanced, metastasized patients divided into high and low risk of death according to the method of the invention. X-axis shows overall survival time in months, Y-axis shows percentage of surviving patients. Patients were discretized into two bins "GAPDH high" and "GAPDH low" with separation at the median. Relative GAPDH level was expressed as "20 - delta CT", wherein delta CT = CT[GAPDH] - CT [RPL37A]. It can be clearly seen that "GAPDH low" patients have a significantly better survival than "GAPDH high" patients (p=0.4%).

Figure 4 shows a Kaplan-Meier plot showing metastasis free survival time of advanced metastasized patients divided into high, intermediate, and low risk of death according to the method of the invention. X-axis shows overall survival time in months, Y-axis shows percentage of surviving patients. Patients were discretized into three bins "GAPDH high", "GAPDH intermediate" and "GAPDH low" with separation at the terciles. Relative GAPDH level was expressed as "20 - delta CT", wherein delta CT = CT [GAPDH] - CT [RPL37A] . It can be clearly seen that "GAPDH low" patients have a significantly better survival than "GAPDH high" patients (p=0.6%).

In experiments to identify the best housekeeping gene mixtures to be used as normalization genes for breast cancer prognosis or prediction, it was surprisingly found that mixtures of primers and probes of different housekeepers perform similar to separate measurements of the single genes by qPCR. For example, primers and probes where mixed for four housekeeping genes into one PCR well (ACTG1, CALM2, OAZ1 und PPIA). With one fourth of the concentration of each single reaction (1+1+1+1) it was surprisingly found that the cycle threshold value of the mean of single reactions was nearly identically to the measured cycle threshold value of the mixed reaction.

Single housekeeping genes in the mixture can also be weighted in the metaplexing reaction by raising the relative amount of primers and probes compared to other genes in the mixture. Mastermix, enzymes, MgS04 are present in standard concentrations or are adjusted for optimized primer and probe performances.

Table 6 shows the groups of probes used for a plurality of housekeeping genes, which are designated as housekeeping gene mixtures. The mixture includes respective forward and reverse primer and qPCR-Probe.

**Table 6: Housekeeping Gene Mixtures, HKM:**

| **Housekeeper Mixture Name** | **Probes in Mixture** |
|---|---|
| HKM | R117 + R113 + BC268 + R115 |
| HKM-1 | R113 + BC268 + R115 |
| HKM-2 | R117 + BC268 + R115 |
| HKM-3 | R117 + R113 + R115 |
| HKM-4 | R117 + R113 + BC268 |
| HKM1246 | R117 + R113 + R115 + R16 |

Experiments showed that using the housekeeping gene mixtures (HKM) listed in table 6 produced consistently superior results as opposed to using single housekeeping genes as internal reference (data not shown). In each HKM the same detectable label (a FAM/TAMRA labeled probe) was used, thus producing a combined detectable signal. This combined signal was a more stable signal and thus better suited to be used as internal control

To find an optimal housekeeping gene or mixture we propose a supervised approach that assigns each gene its optimal housekeeper or mixture.

In order to determine which house keeping gene or housekeeping gene mixture is best suited for statistical analysis and allows the best separation of patients with respect to expression of a gene of interest (GOI), separation was performed by using an algorithm, e.g. correlation coefficient, fisher score or t-test.

For each gene the (at least one further)housekeeper is selected in a way that, by subtracting its CT-value vector from the gene's CT-value vector, maximizes the information content of that gene with respect the output vector (here the output vector is a step function, '1' for positive patients and '0' for control patients). As a measure for the information content we use 3 criteria, correlation coefficient, Fisher score and t-test.

The approach was tested under a 100 times 10-fold cross validation setting to account for the stability of housekeeper-assignments. Combined housekeeping gene mixtures, as listed in table 6, are chosen much more frequently than individual housekeeping genes (data not shown).

## Claims

1. A method for predicting an outcome of a patient suffering from or at risk of developing breast cancer, said method comprising the steps of:
a) obtaining a biological sample from said patient;
b) quantifiably determining the gene expression level of GAPDH in relation to RPL37A;
c) arriving at a prediction by means of a mathematical discriminant function of the expression level of GAPDH in relation to RPL37A.

2. Method of claim 1, wherein the mathematical discriminate function comprises classifying the sample into one of at least two categories.

3. Method of claim 1 or 2, wherein the mathematical discriminate function is a comparison with one, two or more predetermined threshold values.

4. Method of claim 2 wherein the two categories are indicative of a high risk or low risk of developing metastasis.

5. Method of any of the preceding claims, wherein the expression level is determined by
a) a hybridization-based method;
b) a PCR-based method;
c) determining the protein level,
d) a method based an the electrochemical detection of GAPDH and RPL37A molecules, and/or by
e) an array-based method.

6. Method of any of the preceding claims, **characterized in that** the expression level of GAPDH and RPL37A is determined with RT-PCR of the respective mRNAs.

7. Method of any of the preceding claims, **characterized in that** the expression level GAPDH and RPL37A is determined in formalin and/or paraffin fixed tissue samples.

8. Method of any of the preceding claims, wherein, after lysis, the samples are treated with silica-coated magnetic particles and a chaotropic salt, in order to purify the nucleic acids contained in said sample for further determination.

9. Use of a nucleic acid for performing the method according to any of claims 1 to 8, said nucleic acid being selected from the group consisting of
a) SEQ ID NO: 1, SEQ ID NO: 2, SEQ ID NO: 7, SEQ ID NO: 8, SEQ ID NO: 13, SEQ ID NO: 14, SEQ ID NO: 19, SEQ ID NO: 20, and fractions, fragments, complements, hybridizing counterparts thereof, or molecules sharing a sequence identity of at least 70%, preferably 95%,
b) a hybridizing counterpart of a nucleic acid according to one of SEQ ID NO:25 and SEQ ID NO: 26,
c) a nucleic acid complementary to a fragment of at least 15 contiguous nucleotides of one of SEQ ID NO:25 and SEQ ID NO: 26; and
d) a nucleic acid comprising a sequence of at least 15 contiguous nucleotides of one of SEQ ID NO:25and SEQ ID NO: 26.

## Patentansprüche

1. Verfahren zur Vorhersage eines Ergebnissens für einen Patienten, der an Brustkrebs leidet oder bei dem die Gefahr besteht, dass er Brustkrebs entwickelt, wobei das Verfahren die folgenden Schritte umfasst:
a) Gewinnen einer biologischen Probe von dem Patienten;
b) quantifizierbares Bestimmen des Genexpressionsniveaus von GAPDH relativ zu RPL37A;
c) Erhalten einer Prognose mittels einer mathematischen Diskriminationsfunktion des Expressionsniveaus von GAPDH relativ zu RPL37A.

2. Verfahren gemäß Anspruch 1, wobei die mathematische Diskriminationsfunktion das Klassifizieren der Probe in eine von wenigstens zwei Kategorien umfasst.

3. Verfahren gemäß Anspruch 1 oder 2, wobei die mathematische Diskriminationsfunktion ein Vergleich mit einem, zwei oder mehr vorbestimmten Schwellenwerten ist.

4. Verfahren gemäß Anspruch 2, wobei die zwei Kategorien anzeigen, ob ein hohes Risiko oder ein geringes Risiko der Entwicklung von Metastasen besteht.

5. Verfahren gemäß einem der vorstehenden Ansprüche, wobei das Expressionsniveau bestimmt wird durch
a) ein auf Hybridisierung beruhendes Verfahren;
b) ein auf PCR beruhendes Verfahren;
c) Bestimmen der Proteinkonzentration;
d) ein Verfahren, das auf dem elektrochemischen Nachweis von GAPDH-und RPL37A-Molekülen beruht, und/oder
e) ein auf einem Mikroarray beruhendes Verfahren.

6. Verfahren gemäß einem der vorstehenden Ansprüche, **dadurch gekennzeichnet, dass** das Expressionsniveau von GAPDH und RPL37A mit RT-PCR der jeweiligen mRNAs bestimmt wird.

7. Verfahren gemäß einem der vorstehenden Ansprüche, **dadurch gekennzeichnet, dass** das Expressionsniveau von GAPDH und RPL37A in formalin-und/oder paraffinfixierten Gewebeproben bestimmt wird.

8. Verfahren gemäß einem der vorstehenden Ansprüche, wobei die Proben nach einer Lyse mit Siliciumdioxid-beschichteten magnetischen Teilchen und einem chaotropen Salz behandelt werden, um die in der Probe enthaltenen Nucleinsäuren für die weitere Bestimmung zu reinigen.

9. Verwendung einer Nucleinsäure zur Durchführung des Verfahrens gemäß einem der Ansprüche 1 bis 8, wobei die Nucleinsäure aus der Gruppe ausgewählt ist, die aus Folgenden besteht:
a) SEQ ID Nr. 1, SEQ ID Nr. 2, SEQ ID Nr. 7, SEQ ID Nr, 8, SEQ ID Nr. 13, SEQ ID Nr. 14, SEQ ID Nr. 19, SEQ ID Nr. 20 und Fraktionen, Fragmenten, Komplementen, hybridisierenden Gegenstücken davon oder Molekülen mit einer Sequenzidentität von wenigstens 70%, vorzugsweise 95%.
b) einem hybridisierenden Gegenstück einer Nucleinsäure gemäß SEQ ID Nr. 25 oder SEQ ID Nr. 26;
c) einer Nucleinsäure, die zu einem Fragment von wenigstens 15 aufeinanderfolgenden Nucleotiden von SEQ ID Nr. 25 oder SEQ ID Nr. 26 komplementär ist; und
d) einer Nucleinsäure, die eine Sequenz von wenigstens 15 aufeinanderfolgenden Nucleotiden von SEQ ID Nr. 25 oder SEQ ID Nr. 26 umfasst.

## Revendications

1. Procédé de prédiction de l'évolution de l'état de santé d'un patient atteint d'un cancer du sein ou risquant de le développer, ledit procédé comprenant les étapes consistant à :
a) obtenir un échantillon biologique dudit patient ;
b) déterminer de façon quantifiable le niveau d'expression génique de GAPDH par rapport à PRL37A ;
c) arriver à une prédiction au moyen d'une fonction discriminante mathématique du niveau d'expression de GAPDH dans la PRL37A.

2. Procédé selon la revendication 1, dans lequel la fonction discriminante mathématique comprend la classification de l'échantillon dans une des au moins deux catégories.

3. Procédé selon la revendication 1 ou 2, dans lequel la fonction discriminante mathématique est une comparaison avec une, deux valeurs seuil prédéterminées ou plus.

4. Procédé selon la revendication 2, dans lequel les deux catégories indiquent un risque élevé ou un faible risque de développer des métastases.

5. Procédé selon l'une quelconque des revendications précédentes, dans lequel le niveau d'expression est déterminé par
a) un procédé basé sur l'hybridation :
b) un procédé à base d'amplification en chaîne par polymérase ;
c) la détermination du niveau de protéines,
d) un procédé basé sur la détection électrochimique de molécules GAPDH et PRL37A, et/ou par
e) un procédé à base de tableau.

6. Procédé selon l'une quelconque des revendications précédentes, **caractérisé en ce que** le niveau d'expression de GAPDH et de PRL37A est déterminé avec une amplification en chaîne par polymérase en temps réel des ARNm respectifs.

7. Procédé selon l'une quelconque des revendications précédentes, **caractérisé en ce que** le niveau d'expression de GAPDH et de PRL37A est déterminé dans des échantillons de tissu fixés à la formaline ou à la paraffine.

8. Procédé selon l'une quelconque des revendications précédentes, dans lequel, après lyse, les échantillons sont traités avec des particules magnétiques revêtues de silice et un sel chaotropique, afin de purifier les acides nucléiques contenus dans ledit échantillon pour une détermination supplémentaire.

9. Utilisation d'un acide nucléique pour réaliser le procédé selon l'une quelconque des revendications 1 à 8, ledit acide nucléique étant choisi dans le groupe constitué par :
a) SEQ ID N° : 1, SEQ ID N° : 2 SEQ ID N° 7, SEQ ID N° : 8, SEQ ID N° : 13, SEQ ID N° :14, SEQ ID N° :19, SEQ ID N° : 20, et leurs fractions, fragments, compléments, et équivalents d'hybridation, ou des molécules partageant une identité de séquence d'au moins 70 %, de préférence 95 %,
b) un équivalent d'hybridation d'un acide nucléique selon une de SEQ ID N° : 25 et SEQ ID N° : 26,
c) un acide nucléique complémentaire à un fragment d'au moins 15 nucléotides continus de l'un de SEQ ID N° : 25 et SEQ ID N° : 26 ; et
d) un acide nucléique comprenant une séquence d'au moins 15 nucléotides continus de l'un de SEQ ID N° : 25 et SEQ ID N° : 26.


### SEQUENCE LISTING

<110> Siemens Medical Solutions Diagnostics Gmbh <120> Molecular Marker for Cancer Prognosis <130> 200720887
<160> 26
<170> PatentIn version 3.3
<210> 1
   <211> 28
   <212> DNA
   <213> Artificial
<220>
   <223> primer/probe
<400> 1
   aaggtgaagg tcggagtcaa cggatttg 28
<210> 2
   <211> 18
   <212> DNA
   <213> Artificial
<220>
   <223> primer/probe
<400> 2
   tggctggcgg tgcctgga 18
<210> 3
   <211> 20
   <212> DNA
   <213> Artificial
<220>
   <223> primer/probe
<400> 3
   aggcccccct gaaccccaag 20
<210> 4
   <211> 24
   <212> DNA
   <213> Artificial
<220>
   <223> primer/probe
<400> 4
   tggttggatg gcaagcatgt ggtg 24
<210> 5
   <211> 22
   <212> DNA
   <213> Artificial
<220>
   <223> primer/probe
<400> 5
   tcgcgtctcg gaaaccggta gc 22
<210> 6
   <211> 23
   <212> DNA
   <213> Artificial
<220>
   <223> primer/probe
<400> 6
   tgcttccaca agaaccgcga gga 23
<210> 7
   <211> 17
   <212> DNA
   <213> Artificial
<220>
   <223> primer/probe
<400> 7
   gccagccgag ccacatc 17
<210> 8
   <211> 21
   <212> DNA
   <213> Artificial
<220>
   <223> primer/probe
<400> 8
   tgtggttcct gcatgaagac a 21
<210> 9
   <211> 22
   <212> DNA
   <213> Artificial
<220>
   <223> primer/probe
<400> 9
   ctggcaccac accttctaca ac 22
<210> 10
   <211> 22
   <212> DNA
   <213> Artificial
<220>
   <223> primer/probe
<400> 10
   tttcatctgc actgccaaga ct 22
<210> 11
   <211> 20
   <212> DNA
   <213> Artificial
<220>
   <223> primer/probe
<400> 11
   gagcgagctg agtggttgtg 20
<210> 12
   <211> 20
   <212> DNA
   <213> Artificial
<220>
   <223> primer/probe
<400> 12
   cgagccgacc atgtcttcat 20
<210> 13
   <211> 16
   <212> DNA
   <213> Artificial
<220>
   <223> primer/probe
<400> 13
   ccaggcgccc aatacg 16
<210> 14
   <211> 24
   <212> DNA
   <213> Artificial
<220>
   <223> primer/probe
<400> 14
   gtgacagcgg aagtggtatt gtac 24
<210> 15
   <211> 30
   <212> DNA
   <213> Artificial
<220>
   <223> primer/probe
<400> 15
   caaacataat ctgagtcatc ttctctctgt 30
<210> 16
   <211> 24
   <212> DNA
   <213> Artificial
<220>
   <223> primer/probe
<400> 16
   tattcatgcc ttctttcact ttgc 24
<210> 17
   <211> 21
   <212> DNA
   <213> Artificial
<220>
   <223> primer/probe
<400> 17
   agtcagttgg tcagccatgc t 21
<210> 18
   <211> 21
   <212> DNA
   <213> Artificial
<220>
   <223> primer/probe
<400> 18
   aagcccaaaa agctgaaggt t 21
<210> 19
   <211> 80
   <212> DNA
   <213> Artificial
<220>
   <223> PCR Product
<400> 19
   gccagccgag ccacatcgct cagacaccat ggggaaggtg aaggtcggag tcaacggatt 60
   tggtcgtatt gggcgcctgg 80
<210> 20
   <211> 65
   <212> DNA
   <213> Artificial
<220>
   <223> PCR Primer, Probe or Prduct
<400> 20
   tgtggttcct gcatgaagac agtggctggc ggtgcctgga cgtacaatac cacttccgct 60
   gtcac 65
<210> 21
   <211> 119
   <212> DNA
   <213> Artificial
<220>
   <223> PCR Product
<400> 21
   ctggcaccac accttctaca acgagctgcg cgtggccccg gaggagcacc cagtgctgct 60
   gaccgaggcc cccctgaacc ccaaggccaa cagagagaag atgactcaga ttatgtttg 119
<210> 22
   <211> 77
   <212> DNA
   <213> Artificial
<220>
   <223> PCR Product
<400> 22
   tttcatctgc actgccaaga ctgagtggtt ggatggcaag catgtggtgt ttggcaaagt 60
   gaaagaaggc atgaata 77
<210> 23
   <211> 72
   <212> DNA
   <213> Artificial
<220>
   <223> PCR Product
<400> 23
   gagcgagctg agtggttgtg tggtcgcgtc tcggaaaccg gtagcgcttg cagcatggct 60
   gaccaactga ct 72
<210> 24
   <211> 83
   <212> DNA
   <213> Artificial
<220>
   <223> PCR Product
<400> 24
   cgagccgacc atgtcttcat ttgcttccac aagaaccgcg aggacagagc cgccttgctc      60
   cgaaccttca gctttttggg ctt                                              83
<210> 25
   <211> 1310
   <212> DNA
   <213> Homo sapiens
<400> 25
   aaattgagcc cgcagcctcc cgcttcgctc tctgctcctc ctgttcgaca gtcagccgca 60
   tcttcttttg cgtcgccagc cgagccacat cgctcagaca ccatggggaa ggtgaaggtc 120
   ggagtcaacg gatttggtcg tattgggcgc ctggtcacca gggctgcttt taactctggt 180
   aaagtggata ttgttgccat caatgacccc ttcattgacc tcaactacat ggtttacatg 240
   ttccaatatg attccaccca tggcaaattc catggcaccg tcaaggctga gaacgggaag 300
   cttgtcatca atggaaatcc catcaccatc ttccaggagc gagatccctc caaaatcaag 360
   tggggcgatg ctggcgctga gtacgtcgtg gagtccactg gcgtcttcac caccatggag 420
   aaggctgggg ctcatttgca ggggggagcc aaaagggtca tcatctctgc cccctctgct 480
   gatgccccca tgttcgtcat gggtgtgaac catgagaagt atgacaacag cctcaagatc 540
   atcagcaatg cctcctgcac caccaactgc ttagcacccc tggccaaggt catccatgac 600
   aactttggta tcgtggaagg actcatgacc acagtccatg ccatcactgc cacccagaag 660
   actgtggatg gcccctccgg gaaactgtgg cgtgatggcc gcggggctct ccagaacatc 720
   atccctgcct ctactggcgc tgccaaggct gtgggcaagg tcatccctga gctgaacggg 780
   aagctcactg gcatggcctt ccgtgtcccc actgccaacg tgtcagtggt ggacctgacc 840
   tgccgtctag aaaaacctgc caaatatgat gacatcaaga aggtggtgaa gcaggcgtcg 900
   gagggccccc tcaagggcat cctgggctac actgagcacc aggtggtctc ctctgacttc 960
   aacagcgaca cccactcctc cacctttgac gctggggctg gcattgccct caacgaccac 1020
   tttgtcaagc tcatttcctg gtatgacaac gaatttggct acagcaacag ggtggtggac 1080
   ctcatggccc acatggcctc caaggagtaa gacccctgga ccaccagccc cagcaagagc 1140
   acaagaggaa gagagagacc ctcactgctg gggagtccct gccacactca gtcccccacc 1200
   acactgaatc tcccctcctc acagttgcca tgtagacccc ttgaagaggg gaggggccta 1260
   gggagccgca ccttgtcatg taccatcaat aaagtaccct gtgctcaacc 1310
<210> 26
   <211> 434
   <212> DNA
   <213> Homo sapiens
<400> 26
   acttccgctc gtccgcctaa taccgcgcct gcgcaccgcg tctcttcctt tctgggctcg     60
   gacctaggtc gcggcgacat ggccaaacgt accaagaaag tcgggatcgt cggtaaatac    120
   gggacccgct atggggcctc cctccggaaa atggtgaaga aaattgaaat cagccagcac    180
   gccaagtaca cttgctcttt ctgtggcaaa accaagatga agagacgagc tgtggggatc    240
   tggcactgtg gttcctgcat gaagacagtg gctggcggtg cctggacgta caataccact    300
   tccgctgtca cggtaaagtc cgccatcaga agactgaagg agttgaaaga ccagtagacg    360
   ctcctctact ctttgagaca tcactggcct ataataaatg ggttaattta tgtaacaaaa    420
   aaaaaaaaaa aaaa                                                      434